# EUROPEAN PATENT APPLICATION

(11) **EP 2 412 247 A1**
(43) Date of publication of application: **01.02.2012**
(21) Application number: 09842243.9
(22) Date of filing: 26.03.2009
(51) Int. Cl.: A23F 5/18, A23L 3/3472

(54) **METHOD OF OBTAINING ANTIOXIDANT FROM ROASTED COFFEE BEANS, THE ANTIOXIDANT THUS OBTAINED AND FOOD CONTAINING THE SAME**

(71) Applicant: Ajinomoto General Foods, Inc., Shinjuku-ku Tokyo 163-1440 (JP); Kraft Foods Global Brands LLC, Northfield, IL 60093 (US)
(72) Inventor: OZAKI, Kazuto, Tokyo 169-0073 (JP); FUJII, Shigeyoshi, Tokyo 169-0073 (JP)
(74) Representative: Setna, Rohan P.
(86) International application number: PCT/JP2009/056120
(87) International publication number: WO 2010/109629

(57) **Abstract**

Provided is an antioxidant originating in coffee beans. A method which comprises contacting a water extract of roasted coffee or an aqueous solution of the extract with an adsorbent and then eluting an antioxidant adsorbed by the adsorbent with an organic solvent or an aqueous organic solvent.

## Description

Method of obtaining antioxidant from roasted coffee beans, the antioxidant thus obtained, and food containing the same

### Technological Field

The invention concerns an antioxidant component derived from coffee.

### Prior Art

In recent years, it has been recommended that people efficiently consume components high in antioxidants derived from food products as a means of biophylaxis. One means of doing this is to take supplements containing antioxidant components, but it is preferable to obtain them more easily from the foods and beverages commonly consumed. There are various high-antioxidant foods available, but they are still insufficient from the standpoint of antioxidant capacity. There is a demand for the development of methods for identifying components of commonly consumed foods and beverages and supplying them in a convenient manner.

As coffee is a widely-consumed everyday beverage, if it had strong antioxidant capacity, it could constitute a highly outstanding functional beverage. On the other hand, as coffee is a beverage for highly discriminating tastes, if one adds components that do not originate from coffee in order to increase its antioxidant capacity, such as ascorbic acid, tocopherols, and tea catechins, this would adversely affect the beverage's flavor and image, decreasing its product value. For this reason, when imparting powerful antioxidant properties to coffee by adding components having a strong antioxidant effect, it is preferable to use components derived from coffee. Research into antioxidant components derived from coffee has therefore progressed.

Raw coffee beans contain large amounts of chlorogenic acids such as 5CQA, and they are an ingredient that has attracted attention because of their antioxidant properties as polyphenols. For this reason, the simple method of using raw coffee bean extract has been proposed (Patent H5-59696), but because of the characteristic odor of the raw beans themselves contained in this extract, they make it very difficult to maintain the balanced flavor of the beverage, and as skill is required to add them to blends, the flavor is ruined in many cases. Moreover, attempts have been made to add chlorogenic acid alone in order to avoid the effect of raw beans, but this requires a complex manufacturing process (Japanese Unexamined Patent Application Publication No. H9-143465), and when chlorogenic acids alone are simply added to coffee for common use, the bitter taste inherent in the main component of chlorogenic acid ruins the flavor balance. In order to avoid these negative qualities of raw beans and chlorogenic acid, the method was developed of adding the product of the Maillard reaction, in which, in contrast to the aforementioned ingredients, amino acids and sugar are reacted (Patent No. 3855293), but as this method is also complicated and uses an ingredient not derived from coffee, it also reduces the value of the beverage.

Because of this situation, there has been a search for an ingredient derived from coffee alone, having powerful antioxidant properties, and showing a favorable balance with coffee.
Patent Document 1: Patent No. H5-59696
Patent Document 2: Japanese Unexamined Patent Application Publication No. H9-143465
Patent Document 3: Patent No. 3855293

### Presentation of the Invention

### Problems to be Solved by the Invention

The invention provides a method for obtaining a new antioxidant from coffee, the antioxidant thus obtained, and a coffee beverage containing said antioxidant.

### Means for Solving Problems

The inventors of the present invention conducted extensive research in an effort to solve the above problem, and discovered that roasted coffee extract has potent antioxidant properties and shows a highly favorable balance with the flavor of coffee.

Accordingly, the invention is intended to provide a method for obtaining an antioxidant from an aqueous extract solution of roast coffee in which said aqueous coffee extract solution or an aqueous solution of a solid substance extracted from coffee is brought into contact with an adsorbing agent, and the antioxidant adsorbed to said adsorbing agent is eluted using an organic solvent or an aqueous organic solvent.

In a preferred embodiment, the organic solvent or the organic solvent of the aqueous organic solvent is an alcohol, and in a particularly preferred embodiment, said alcohol is ethanol. In a preferred embodiment, said adsorbent is a styrene divinylbenzene polymer or polyvinylpolypyrrolidone.

In a preferred embodiment, the method of the invention comprises a process in which coffee beans roasted at 180-300°C for a period of 1 minute to 1 hour are pulverized, said crushed coffee beans are extracted with boiling water, this extract is freeze-dried, the solid obtained is redissolved in water, the coffee beans used are brought into contact with an amount of styrene divinylbenzene copolymer or polyvinylpolypyrrolidone resin 0.5-200 times the weight of the beans and said antioxidant is adsorbed, the resin is washed with an amount of water 0.5-20 times the volume of the resin, said antioxidant is eluted from the resin first with 30% ethanol and then with 70% ethanol, and it is then combined with the extract solution and concentrated.

The invention also concerns an antioxidant obtained by the above method.

Moreover, the present invention further concerns a food product to which the antioxidant obtained by the above method has been added. In a preferred embodiment, this food product is a coffee beverage, and the antioxidant obtained by the above method is added to the coffee beverage in an amount of 10-200%, and preferably 10-100%, of the coffee solid component originally contained in the coffee beverage.

### Effect of the Invention

One can efficiently consume a substance having high antioxidant capacity from a commonly-used coffee beverage without adversely affecting the flavor balance of said beverage.

### Description of Preferred Embodiments

In the prior art, high-antioxidant-capacity coffee bean extract was derived from chlorogenic acids, mainly 5CQA. Chlorogenic acids are known to react with other components when roasted, decomposing until there is virtually no residue remaining. For this reason, antioxidants derived from coffee have been obtained solely from raw coffee bean extract. However, the inventors of the present invention found that when they fractionated the roast coffee extract, substances having a high antioxidant capacity were contained in the roast coffee separate from the chlorogenic acids. Moreover, in the process of studying methods for fractionating these substances, they successfully isolated substances showing high antioxidant capacity by carrying out adsorption using an adsorbent followed by elution with a particular solvent. As these substances are not contained in raw coffee beans, they are produced by roasting. For this reason, they do not have the characteristic bitter taste of chlorogenic acids or the odor of raw beans, but have a high-quality flavor close to that of roast coffee.

The degree of roasting of the coffee beans used in the invention may be the same as that of ordinary coffee, but one can also freely adjust this degree of roasting depending on the flavor of the target coffee. With respect to the roasting machine as well, there are no particular restrictions on batch size (several 10s of g-tons) or heat source (open flame, hot air, far infrared, charcoal, etc.), and any type of roasting machine may be used. For the beans used as well, species such as arabica, robusta, or liberica may be used, the beans may be blended if necessary, and one may use any method to tailor the taste to the flavor of the target coffee. Roasting may be carried out either before or after blending, and any method that allows the target flavor to be obtained may be used. There are no particular restrictions on the crushing machine used, provided that the granules obtained are suitable for extraction. There is also no need to limit the granule diameter to a particular range.

A simple dripper may be used in the extraction method, but a large-scale counterflow percolator, which provides high efficiency and high concentration capacity for industrial use, is preferred. There is no particular set temperature for extraction, and the antioxidant of the present invention may be extracted using cold water or hot water. In the instant coffee manufacturing process, extraction is frequently carried out in a temperature range that allows polysaccharide hydrolysis in order to increase the recovery rate of soluble components, but as the substance of the invention does not decompose in this temperature range, it is also suitable for such extraction. Another possible method is to extract the antioxidant of the invention at a lower temperature, raise the extraction temperature, and then extract it separately as an ordinary coffee solid. However, extraction is generally carried out at 30-200°C, and preferably 60-180°C, with a temperature of 100-180°C being particularly preferred. The amount of water used in extraction should be 1-30 times, preferably 2-20 times, and even more preferably 3-10 times the weight of the coffee beans.

The adsorbent used should preferably be low cost and allow easy recovery of the antioxidant, with examples including activated carbon, a strong anion-exchange resin, a weak anion-exchange resin, a strong cation-exchange resin, a weak cation-exchange resin, a hydrophobic group-bonded silica adsorbent, methacrylate adsorbent resin, or a styrene divinylbenzene adsorbent resin such as HP-20 or polyvinylpolypyrrolidone (PVPP), with styrene divinylbenzene adsorbent resin and polyvinylpolypyrrolidone being preferred, but there are no particular restrictions. With respect to the resin, one type is sufficient in order to separate the target antioxidant, but a multiple combination may also be used in order to obtain a higher-capacity antioxidant. The amount of the adsorbent used should be 0.1-200 times, preferably 0.2-100 times, and even more preferably 0.5-20 times the weight of the coffee beans used in extraction.

For adsorption, the batch method may be used, but adsorption efficiency is higher if the column method is used, so use of the column method is preferred in order to achieve high yield. In the case of the column method, when pressure loss becomes great, operating efficiency decreases, so the resin particle diameter should preferably be fairly large, with a size of approx. 200-500 microns being preferred with respect to balance with adsorption efficiency, but there are no particular restrictions on this range. Moreover, as operations are conducted by adsorption/elution alone and chromatography separation is not necessary, there is no need to have a large number of column stages, with a column height of about three times the column diameter being sufficient, and it can be made even lower. Elution will be easier if the elution solvent flows from the opposite side during adsorption, but this is not absolutely necessary, and a method suited to the equipment is best.

After adsorption, washing with water is carried out in order to elute the unfavorable components and determine the antioxidant capacity. The amount of water used in washing should be 0.1-200 times, and preferably 1-20 times greater than the weight of the coffee beans.

Any solvent may be used as the elution solvent of the antioxidant, provided it is an organic solvent having a polarity lower than water or an aqueous organic solvent, and solvents may be used individually or in combination. For example, an ester type such as ethyl acetate, an ether type such as ethyl ether, a ketone type such as acetone, or an alcohol type such as ethanol are easy to remove after elution, and these solvents may be used individually or in combination. It is also possible to mix water and an organic solvent miscible in water and use this mixture as an aqueous organic solvent. The percentage of the organic solvent in the aqueous organic solvent should be 1-99%, preferably 5-95%, and particularly preferably 10-90% by volume. 10-90% aqueous ethanol is particularly preferred, and within this range, 30-70% aqueous ethanol, and particularly 50% aqueous ethanol, are easy-to-use solvents because they allow favorable recovery of antioxidants. Methanol is also a suitable solvent for the same reason. However, it shows somewhat poor recovery of the solid component compared to ethanol. The solid component may be used as is as an antioxidant, but it should preferably be dried so as not to contain any ethanol or methanol. Drying may be carried out either by vacuum freeze-drying or spray drying, and the method should be determined taking into account both the quality of the target product and cost. The solid obtained has outstanding flavor either used alone or with coffee, so it can be used as is as instant coffee or diluted to a suitable concentration and made into a liquid coffee beverage, and it may also be blended with other coffees to make instant coffee or liquid coffee. It may also be combined with cream, sweeteners, flavorings, etc. to make a milk coffee drink.

### Working Examples

### Working Example 1

### Obtaining an antioxidant from coffee

Colombian arabica coffee beans were roasted for 5 minutes at 250-260°C and crushed.

500 g of boiling water was added to 50 g of coffee beans, and extraction was carried out using a paper filter. The extracted solution cooled rapidly. The extraction operation was repeated 10 times and 3.4 kg of extract solution was obtained. It was centrifuged at 5000 rpm for 10 minutes and the supernatant was collected. The supernatant was filtered through the Whatman glass fiber filter GF/B, and the filtrate was vacuum freeze-dried. 98 g of dried solid component was obtained.

270 g of water was added to 30 g of the dried solid component to obtain test solution, and the solution was adsorbed to 500 mL of styrene divinylbenzene copolymer resin HP-20 (Mitsubishi Chemical) charged in a glass column of internal diameter 40 mm and length 750 mm. The pigment and sugar showing low antioxidant capacity were eluted with 1000 mL of water, after which the adsorbed component was eluted with 1000 mL each or 30% and 70%.

The 30% ethanol elution solution and the 70% ethanol elution solution were combined, they were concentrated under reduced pressure, the ethanol was removed, and vacuum freeze-drying was carried out to obtain 9.2 g of the final solid component. The aqueous elution solution obtained before elution with 30% ethanol was also vacuum freeze-dried to obtain 20.5 g of solid component.

### Working Example 2

### Measurement of antioxidant capacity

Antioxidant capacity was measured by the ORAC (Oxygen Radical Absorbance Capacity) method. A specified amount of dried sample was dissolved/adjusted to constant volume with a 50/48/2 mixture of acetone/water/acetic acid, and this was taken as the test stock solution. The stock solution was subjected to serial dilution to obtain test solutions.

Measurement of the test solutions was conducted by adding the fluorescent probe fluorescein to Trolox standard solution, incubating the mixture, adding the radical initiator AAPH (2,2'-azo-bis(2-amidinopropane)dihydrochloride), and conducting measurements at an excitation wavelength of 485 nm and a measurement wavelength of 528 nm. ORAC values were measured by calculating equivalent volumes based on area values.

The ORAC values (U/g) for the extracted solid component, aqueous extracted solid component, and ethanol-extracted solid component of Working Example 1 are shown in the Table below. The ethanol-extracted solid component was found to show strong antioxidant capacity.

**[Table 1]**

| | ORAC (U/g) |
|---|---|
| Extracted solid component of Working Example 1 | 2500 |
| Aqueous extracted component of Working Example 1 | 900 |
| Ethanol-extracted component of Working Example 1 | 6200 |

### Working Example 3

### Flavor evaluation (1)

The final solid component of Working Example 1 was added to instant coffee and the effect on flavor was evaluated. In order to strengthen the antioxidant capacity, we used raw coffee bean hot water extract and chlorogenic acid, and conducted comparisons with these substances.

**[Table 2]**

| Test item no. | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| Instant coffee | 1.5 | 1.5 | 1.5 | 1.5 | 1 | | 1.5 | 1.5 | 1.5 | 1.5 | 1.5 | 1.5 | 1.5 | 1.5 | 1.5 |
| Product of the invention | | 0.2 | 0.5 | 1 | 1 | 1 | | | | | | | | | |
| Raw bean hot water extract | | | | | | | 0.2 | 0.5 | 1 | | | | | | |
| Raw bean | | | | | | | | | | 0.15 | 0.4 | 0.75 | | | |
| ethanol | | | | | | | | | | | | | | | |
| extract | | | | | | | | | | | | | 0.1 | 0.25 | 0.5 |
| 5CQA | | | | | | | | | | | | | | | |
| CRAC | 3600 | 4840 | 6700 | 9800 | 8600 | 6200 | 4800 | 6600 | 9600 | 4800 | 6800 | 9600 | 4800 | 6600 | 9600 |
| Flavor | 4 | 5 | 5 | 4 | 5 | 4 | 4 | 3 | 2 | 4 | 3 | 2 | 4 | 2 | 1 |

| | | | | | | | | | | | | | | | |
|---|---|---|---|---|---|---|---|---|---|---|---|---|---|---|---|
| 1 Poor 3 Ordinary 5 Good | | | | | | | | | | | | | | | |

The final solid component of Working Example 1 had an outstanding aroma both alone and with coffee, and its compatibility when added to coffee was outstanding. The conventional coffee bean extract had a fishy aroma and the characteristic bitter taste of chlorogenic acid, adversely affecting the flavor, but none of these problems were seen with the antioxidant of the invention, and it can therefore be said to be an outstanding antioxidant.

### Working Example 4

### Flavor Evaluation (2)

The final solid component obtained in Working Example 1 was added together with commercial sugar to liquid coffee, and flavor was evaluated.

**[Table 3]**

| Test item no. | 1 | 2 | 3 | 4 | 5 |
|---|---|---|---|---|---|
| Commercial coffee with sugar added | 100 | 100 | 100 | 100 | 100 |
| Milk | 100 | 100 | 100 | 100 | 100 |
| Product of the invention | | 0.1 | 0.5 | 1 | 2 |
| Flavor | 3 | 4 | 5 | 5 | 4 |

| | | | | | |
|---|---|---|---|---|---|
| 1 Poor 3 Ordinary 5 Good | | | | | |

The final solid component of Working Example 1 did not impair the flavor of liquid coffee when added to it. Moreover, it was also confirmed to show good compatibility with milk and sugar. Accordingly, the product of the invention can be used in both black liquid coffee and liquid coffee products with milk and sugar added.

### Working Example 5

### Obtaining an antioxidant from coffee

Colombian arabica coffee was roasted for 5 min. at 250-260°C and crushed before use. 500 g of boiling water was added to 50 g of coffee beans, and extraction was carried out using a paper filter. The extracted solution cooled rapidly. The extraction operation was repeated 5 times and 3.5 kg of extract solution was obtained. It was centrifuged at 5000 rpm for 10 minutes and the supernatant was collected. The supernatant was filtered through the Whatman glass fiber filter GF/B, and the filtrate was vacuum freeze-dried. 50 g of dried solid component was obtained. 190 mL of water was added to 10 g of the solid component and dissolved.

400 mL of polyvinylpolypyrrolidone resin was suspended in of water, and after it had retained sufficient water, the fine powder was removed by decanting. A glass column having an internal diameter of 40 mm and a length of 300 mm was filled with 200 mL of a polyvinylpolypyrrolidone resin slurry, and the test solution was loaded. Elution was carried out with 200 mL of water, followed by 4 solvents: ethanol, 50% ethanol, methanol, and 50% methanol, and a comparison test was conducted.

**[Table 4]**

| | Ethanol | 50% | Methanol | 50% |
|---|---|---|---|---|
| | | ethanol | | methanol |
| Amount recovered (g) | 1.5 | 2 | 1.2 | 1.6 |
| ORAC(U/g) | 8000 | 8200 | 7600 | 7600 |

The solvents were removed from the various elution fractions under reduced pressure, and vacuum freeze-drying was carried out. Table 5 shows the amounts of antioxidants recovered and their antioxidant capacity. All of them showed strong capacity.

**[Table 5]**

| | Solid component | ORAC |
|---|---|---|
| Working Example 1 | 3.0 | 6200 |
| Aqueous extract | 2.1 | 3000 |
| Ethanol extract | 0.9 | 12500 |

### Working Example 6

### Obtaining an antioxidant from coffee

97 g of water was added and dissolved in 3 g of the final solid component of Working Example 1 obtained by adsorption with the styrene divinylbenzene copolymer resin HP-20. 200 mL of polyvinylpolypyrrolidone resin was suspended in water until it retained sufficient water, and the fine powder was removed by decanting. A glass column having an internal diameter of 40 mm and a length of 300 mm was filled with 100 mL of a polyvinylpolypyrrolidone resin slurry, and the test solution was loaded. Elution was carried out with 100 mL of water and 500 mL of 50% ethanol. The aqueous elution fraction was subjected to vacuum freeze-drying as is, and the ethanol was removed from the 50% ethanol elution fraction under reduced pressure, and it was then vacuum freeze-dried. 0.9 g of ethanol-eluted solid substance was obtained. This ethanol-eluted solid substance had an ORAC value of 12,500 U/g, showing an extremely strong antioxidant capacity.

### Possibilities for Industrial Use

As the novel antioxidant of the invention is derived from coffee beans, it provides a high-antioxidant-capacity functional coffee beverage without impairing the flavor of the coffee.

## Claims

1. A method for obtaining an antioxidant from an aqueous extract solution of roast coffee in which said aqueous coffee extract solution or an aqueous solution of an extracted solid substance is brought into contact with an adsorbing agent, and the antioxidant adsorbed to said adsorbing agent is eluted using an organic solvent or an aqueous organic solvent.

2. The method as claimed in claim 1, in which said organic solvent or organic solvent of an aqueous organic solvent is alcohol.

3. The method as claimed in claim 2, in which said alcohol is ethanol.

4. The method as claimed in any one of claims 1-3, in which said adsorbing agent is styrene divinylbenzene copolymer, polyvinylpolypyrrolidone, or a mixture thereof.

5. The method for obtaining an antioxidant from an aqueous extract solution of roast coffee, in which the aqueous extracted solid of the roast coffee is brought into contact with a styrene divinylbenzene copolymer or polyvinylpolypyrrolidone resin in an amount 0.1-200 times the weight of the coffee beans used, said antioxidant substance is adsorbed, the resin is washed with an amount of water 0.5-20 times the volume of the resin, said antioxidant is eluted from the resin with 30% ethanol and then 70% ethanol, and said elution solution is then combined and concentrated.

6. An antioxidant obtained by any one method as claimed in claims 1-4

7. An antioxidant obtained by the method as claimed in claim 5.

8. A food product having the substance as claimed in claim 6 or claim 7 added.

9. The food product as claimed in claim 8, with said food product being a coffee beverage, in which said antioxidant is added in an amount of 10-200% of the coffee solid component originally contained in said coffee beverage.
